# EUROPEAN PATENT APPLICATION

(11) **EP 1 900 346 A1**
(43) Date of publication of application: **19.03.2008**
(21) Application number: 06425641.5
(22) Date of filing: 18.09.2006
(51) Int. Cl.: A61F 2/46

(54) **Device for filling bone cavities with fluid cement, acetabular cavities in particular**

(71) Applicant: Tecres S.P.A., 37066 Sommacampagna (VR) (IT)
(72) Inventor: Faccioli, Giovanni, 46040 Monzambano (MN) (IT); Soffiatti, Renzo, 37054 Nogara (IT)
(74) Representative: Feltrinelli, Secondo Andrea

(57) **Abstract**

The device (1) for filling bone cavities (C) with fluid cement (F), acetabular cavities in particular, includes a channel (2) for transferring the fluid cement with a first opening (3) which can be associated to an instrument (S) for dispensing the fluid cement and a second opening (4) through which the fluid cement exits and which is associated to an applicator element (6) of fluid to a bone cavity.
The applicator element features a useful dispensing surface (8) that is larger than the transfer channel.

## Description

The present invention refers to a device for filling bone cavities with fluid cement, acetabular cavities in particular.

As is common knowledge, arthroplasty requiring the implant of a prosthetic device to a bone or joint of human body, normally needs a certain quantity of cement (acrylic resins or the like) to keep the prosthesis permanently in place.

In the many different methods, bone cement is injected using a syringe which can be mounted on a dispensing gun containing the fluid cement and fitted with a needle, a rigid cannula - or the like - for its application.

After having inserted the free end of the cannula inside the cavity that needs filling, the fluid cement is injected directly into it with the syringe.

While this is a practical and functional way to fill-in femoral, humeral and vertebral cavities or the like, this method is found to be inefficient if bone cement needs to be injected into acetabular cavities for the subsequent application of hip prosthesis.

For this type of intervention, the spherical head of the hip prosthesis has to be fitted inside the acetabular cavity of the patient, interposing a layer of fluid cement which, once hardened, becomes the bed for the prosthesis joint.

As a rule this method requires the work of a medical operator who manually shapes the mass of cement at the doughy state and fits it inside the acetabular cavity.

The cement mass is then compacted inside the cavity with special hammering instruments, with the hammering side shaped like a dome.

Thanks to the particular shape of the hammering instruments the mass of cement is modelled to form a substantially spherically shaped niche.

This known method does, however, have a few drawbacks.

The fact that the mass of cement is handled manually, for instance, exposes the medical operator to direct contact with potentially toxic and reactive substances.

Moreover, exposure of the cement to the external environment can easily compromise its sterility, rendering it a dangerous vehicle for transmitting infections to the patient undergoing the operation.

It is also necessary to stress the fact that this operation is rather awkward and tiring both for the medical operator and the patient.

The main aim of this invention is to create a device for filling bone cavities with fluid cements, acetabular cavities in particular, allowing to introduce bone cement under optimally sterile and safe conditions for the medical operator who is doing the job and for the patient who is undergoing the operation.

A further purpose of this invention is to be able to fill bone cavities with fluid cement quickly and easily without any serious difficulties for the medical operator or great discomfort for the patient.

Yet another purpose of this invention is to devise a tool that will overcome the above mentioned drawbacks of the well known technique, with a simple, rational and cost-effective solution.

These purposes are all achieved by this device for filling bone cavities with fluid cement, acetabular cavities in particular, including at least one transfer channel for transferring the fluid cement with at least one first opening associated to a dispenser instrument for dispensing said fluid cement and one second opening through which the fluid cement exits, characterised by the fact that it comprises at least one applicator element for filling the bone cavity with fluid cement which is associated to said transfer channel situated by the second opening and which has a useful dispensing surface that is larger than the transfer channel.

Further characteristics and advantages of this invention will appear even more evident from the detailed description of a preferred, but not exclusive, form of embodiment of a device for filling bone cavities with fluid cement, acetabular cavities in particular, illustrated by way of non limiting example in the accompanying drawings, wherein:

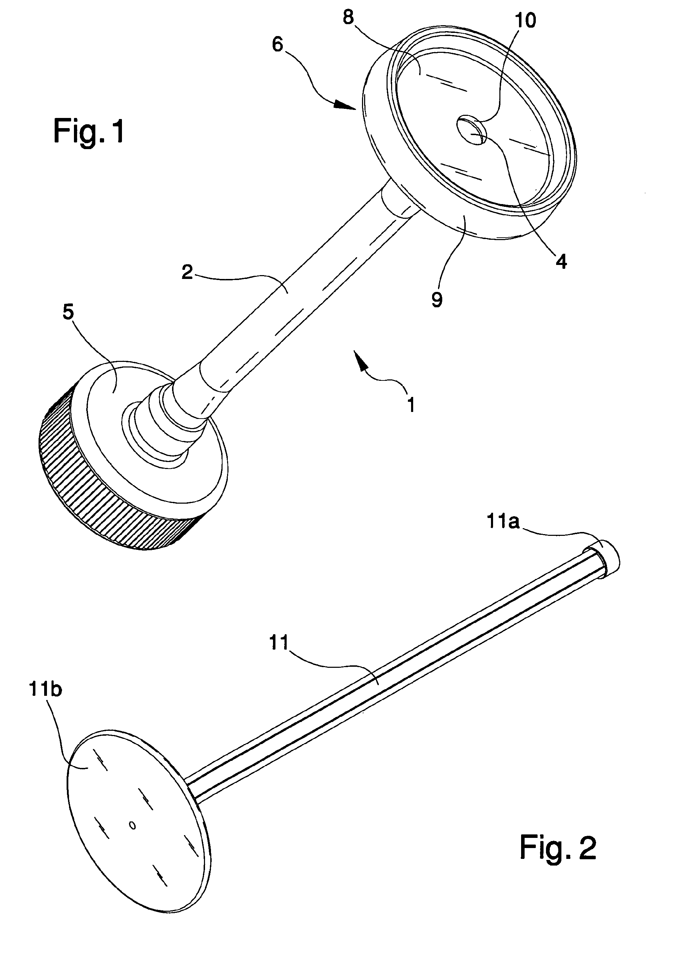

figure 1 is a perspective view of the transfer channel and applicator element as contemplated by the device according to the invention;
figure 2 is a perspective view of the thrust piston as contemplated by the device according to the invention;
figure 3 is a side view of the transfer channel and applicator element as contemplated by the device according to the invention, attached to a fluid cement dispensing gun;
figure 4 is a section view, on an enlarged scale, of a part of figure 3 as the fluid cement is being dispensed inside the acetabular cavity;
figure 5 is a section view, on an enlarged scale, of a part of figure 3 as the fluid cement is being compacted inside the acetabular cavity.

With special reference to the above figures, a device for filling bone cavities with fluid cement, acetabular cavities in particular, has been generally designated by reference number 1.

Device 1 comprises a transfer channel 2 for fluid cements F, such as metacrylate resin type bone cements, which can be radiopaque or of other similar substances.

The transfer channel 2 is substantially rectilinear and tubular in shape. It features a first opening 3 obtained at one end of the channel, and a second opening 4 obtained at the opposite end.

The first opening 3 is associable to a dispenser instrument for dispensing the fluid cement F, like for instance a syringe S, which can be fitted on a gun P, of the type already known.

In detail, attachment means 5 for the syringe S are at the end of the transfer channel 2 where the first opening 3 is.

Such attachment means consist of a cylindrical fitting, coaxial to the transfer channel and associable to the syringe S by means of a moveable coupling, which can be the threaded type, fitted, etc.

In practice, once the cylindrical fitting 5 is attached to the body of the syringe S the fluid cement F is pushed along the transfer channel 2 by the gun P and comes out of the second opening 4.

Usefully, an applicator element 6 is associated to the second opening 4 for injecting the fluid cement F inside a bone cavity C, for example of the acetabular bed type inside which hip prosthesis, or the like, are fitted.

In detail, the applicator element 6 has a useful dispensing surface larger than those of the transfer channel 2. By useful dispensing surface we mean the area through which the fluid cement F passes by the channel and applicator element.

The applicator element 6 features a first side 7 for attaching to the transfer channel 2 and a second side 8 for application of the fluid cement F.

The second side 8 features a perimeter containing border 9 for the fluid cement F, which can be placed touching the edge M of the bone cavity C, that is, against the outer edge of the cavity.

In the particular form of embodiment of the invention, the applicator element 6 has a circular disk shaped plate-like body with its main opposing surfaces defining the first and second sides 7 and 8.

The plate-like body 6 features a through hole 10 through which the fluid cement F flows; it can be coupled to the end of the transfer channel 2 which ends with the second opening 4.

In detail, the through hole 10 is made in the plate-like body 6 in a substantially central position and the transfer channel 2 and applicator element 6 are coaxially associated together.

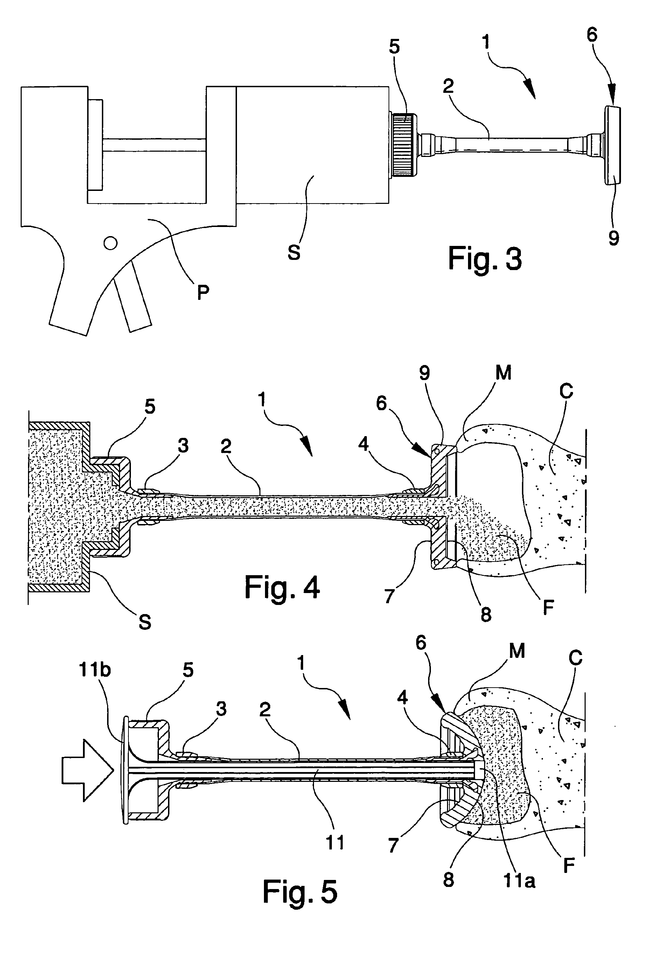

When using, the containing border 9 of the applicator element 6 is placed against the edge M of the bone cavity C so as to isolate the inside of it from the outside and so it can be filled with the fluid cement F without any leaks, as illustrated in figure 4.

In the particular form of embodiment of the invention illustrated in the figures, the containing border 9 consists of a perimeter turned-up edge of the plate-like body 6, extending substantially crossways from the second side 8 of the body itself.

Alternative forms of embodiment are not in anyway excluded, where the applicator element 6 is completely flat and the containing border 9 consists of the edge of the plate-like body 6.

Usefully, the device 1 is fitted with thrusting means 11 that allow the fluid cement F to be pressed along the transfer channel 2 to ensure it is emptied at the end of the operation.

Such thrusting means consist of a longitudinal piston with an end head 11a that can be precisely fitted in the transfer channel 2, and a gripping handle 11b opposite the end head 11a.

After a certain quantity of fluid cement F has been injected into the bone cavity C the syringe S has to be taken off the device 1 and the piston 11 inserted in the transfer channel 2 in order to finish filling the cavity.

The piston 11 is at least as long as the transfer channel 2; in this way, once the piston 11 is inserted in the channel 2 through the first opening 3, the end head 11a reaches the second opening 4 when the gripping handle 11b is against the cylindrical fitting 5.

Advantageously, the applicator element 6 is made in a flexible material while the transfer channel 2 is made in a substantially rigid material; in this way, after the fluid cement F has been dispensed, the device 1 can be used to compress the fluid, making it adhere to the inner walls of the bone cavity C.

By exerting a certain pressure on the transfer channel 2 and on the piston 11 inside it (figure 5), the plate-like body 6 is pressed against the fluid cement F deforming it elastically and turning the fluid cement F into a hollow configuration, suitable for receiving a semi-spherical prosthesis like, for instance, the cotyle of a hip prosthesis.

It should also be pointed out that the rigidity of the transfer channel 2 allows the fluid cement F to be pressurised also when it is being dispensed by the syringe S by simply pressing on the gun P lengthways to the channel itself.

It is also, however, feasible for the transfer channel 2 to have a flexible pipe as an alternative which, when dispensing the fluid cement F using the syringe S, is held manually in position by the bone cavity and, when compacting the fluid cement F in the cavity, becomes rigid when the piston 11 is placed inside it.

It has in fact been found that the described invention achieves the intended purposes.

It should be noted that the particular type of applicator element contemplated in this invention allows the fluid cement to be channelled directly into the bone cavity through the syringe without the risk of unwanted or sudden leaks of fluid or of dangerous contamination.

Also note that with this invention not only is it possible to dispense the fluid cement easily and practically but it can also be compacted quickly either during injection or at the end of dispensing.

The invention thus conceived is susceptible of numerous modifications and variations, all of which falling within the scope of the inventive concept.

Furthermore all the details can be replaced with others that are technically equivalent.

In practice, the materials used, as well as the shapes and dimensions, may be any according to requirements without because of this moving outside the protection scope of the following claims.

## Claims

1. Device for filling bone cavities with fluid cement, acetabular cavities in particular, including at least one transfer channel for transferring the fluid cement with at least one first opening associated to a dispenser instrument for dispensing said fluid cement and one second opening through which the fluid cement exits, **characterised by** the fact that it comprises at least one applicator element for filling the bone cavity with fluid cement which is associated to said transfer channel situated by the second opening and which has a useful dispensing surface that is larger than the transfer channel.

2. Device according to claim 1, **characterized in that** said applicator element comprises a first side for attaching to said transfer channel and a second side for application of said fluid cement.

3. Device according to one or more of the preceding claims, **characterized in that** said second side comprises a containing border for said fluid cement, which can be placed against the edge of said bone cavity.

4. Device according to one or more of the preceding claims, **characterized in that** said applicator element comprises a plate-like body whose main opposing surfaces define said first and second sides.

5. Device according to one or more of the preceding claims, **characterized in that** said plate-like body features a through hole through which said fluid cement flows which can be coupled to said second opening.

6. Device according to one or more of the preceding claims, **characterized in that** said hole is made in said plate-like body in a substantially central position.

7. Device according to one or more of the preceding claims, **characterized in that** said containing border consists of a perimeter turned-up edge of said plate-like body, extending substantially crossways from the body itself.

8. Device according to one or more of the preceding claims, **characterized in that** said plate-like body is substantially circular.

9. Device according to one or more of the preceding claims, **characterized in that** said transfer channel and said applicator element are coaxially associated together.

10. Device according to one or more of the preceding claims, **characterized in that** said applicator element is made in a substantially flexible material.

11. Device according to one or more of the preceding claims, **characterized in that** said transfer channel is made in a substantially rigid material.

12. Device according to one or more of the preceding claims, **characterized in that** said transfer channel comprises attachment means to said dispenser instrument situated by said first opening.

13. Device according to one or more of the preceding claims, **characterized in that** said attachment means comprise at least one fitting substantially coaxial to said transfer channel.

14. Device according to one or more of the preceding claims, **characterized in that** it comprises thrusting means of the fluid cement in said transfer channel.

15. Device according to one or more of the preceding claims, **characterized in that** said thrusting means comprise at least one piston which can be precisely fitted in said transfer channel.

16. Device according to one or more of the preceding claims, **characterized in that** said piston is at least as long as said transfer channel.
